# EUROPÄISCHE PATENTSCHRIFT

(11) **EP 0 937 018 B1**
(45) Veröffentlichungstag und Bekanntmachung des Hinweises auf die Patenterteilung: **29.08.2001**
(21) Anmeldenummer: 97945839.5
(22) Anmeldetag: 15.10.1997
(51) Int. Cl.: C07C 29/14, C07C 33/12, C07C 45/74, C07C 47/225

(54) **VERFAHREN ZUR HERSTELLUNG VON 2-METHYL-4-(2,2,3-TRIMETHYL-CYCLOPENT-3-EN-1-YL)-BUT-2-EN-1-OL**
PROCESS FOR PREPARING 2-METHYL-4-(2,2,3-TRIMETHYL-CYCLOPENT-3-EN-1-YL)-BUT-2-EN-1-OL
PROCEDE DE PREPARATION DE 2-METHYLE-4-(2,2,3-TRIMETHYLE-CYCLOPENT-3-ENE-1-YLE)-BUT-2-ENE-1-OL

(30) Priorität: 24.10.1996 DE 19644250
(43) Veröffentlichungstag der Anmeldung: 25.08.1999
(73) Patentinhaber: Cognis Deutschland GmbH, 40589 Düsseldorf (DE)
(72) Erfinder: MARKERT, Thomas, D-40789 Monheim (DE)
(86) Internationale Anmeldenummer: EP9705689
(87) Internationale Veröffentlichungsnummer: WO9817613

(56) Entgegenhaltungen:
- DE-A- 1 922 391
- CHEMICAL ABSTRACTS, vol. 93, no. 9, 1.September 1980 Columbus, Ohio, US; abstract no. 94886p, Seite 607; XP002056191 & JP 55 036 423 A (TAIYO PERFUMERY) 14.März 1980 in der Anmeldung erwähnt
- CHEMICAL ABSTRACTS, vol. 94, no. 15, 13.April 1981 Columbus, Ohio, US; abstract no. 120971e, Seite 648; XP002056192 & JP 55 139 330 A (TAIYO PERFUMERY) 24.Januar 1980
- K.G. AKAMANCHI, ET AL.: "Truly Catalytic Meerwein-Ponndorf-Verley (MPV) Reduction" TETRAHEDRON LETTERS, Bd. 36, Nr. 28, 10.Juli 1995, OXFORD, GB, Seiten 5085-5088, XP004027744 in der Anmeldung erwähnt
- K.G. AKAMANCHI, ET AL.: "Aluminium Isopropoxide - TFA, a Modified Catalyst for Highly Accelerated Meerwein-Ponndorf-Verley (MPV) Reduction" TETRAHEDRON LETTERS, Bd. 36, Nr. 20, 15.Mai 1995, OXFORD, GB, Seiten 3571-3572, XP004028109 in der Anmeldung erwähnt

## Beschreibung

Die Erfindung betrifft ein verbessertes Verfahren zur Herstellung des Sandel-Riechstoffs 2-Methyl-4-(2,2,3-trimethyl-cyclopent-3-en-1-yl)-but-2-en-1-ol durch Reduktion der entsprechenden Aldehyd-Vorstufe nach Meerwein-Pondorf in Gegenwart basischer Aminverbindungen.

### Stand der Technik

Viele natürliche Riechstoffe stehen, gemessen am Bedarf, in völlig unzureichender Menge zur Verfügung. Aus parfümistischer Sicht besonders geschätzt und wertvoll ist das Sandelholzöl. Es wird durch Wasserdampfdestillation aus dem Kernholz des Sandelbaumes gewonnen, eines tropischen Halbparasiten, der in Indien und Malaysia vorkommt. Kernholz erscheint nach etwa zehn Jahren und beginnt erst bei zwanzigjährigen Bäumen, sich rascher auszubilden. Voll ausgewachsene Bäume werden im Alter von 30 bis 60 Jahren ausgerodet, da die Wurzeln besonders reich an wohlriechendem Kernholz sind [vergl. E.T.Morris, Dragoco Report 1983 (30), 40]. Es ist daher verständlich, daß die Riechstoff-Forschung ständig bemüht ist, geeignete Substitute für natürliches Sandelholzöl zu entwickeln.

Die Schwerpunkte bei der Entwicklung geeigneter Substitute für natürliches Sandelholzöl hat R.E.Naipawer in einem Review skizziert [in: B.M.Lawrence, B.D.Mookherjee, B.J.Willis (Hrsg.): "Flavors and Fragrances: A World Perspective"; Elsevier Publishers, Amsterdam 1988]. In diesem Review ist unter anderem dargestellt, daß seit Mitte der 70-er Jahre Campholenylderivate eine wichtige Rolle unter den synthetisch hergestellten Riechstoffen mit Sandelduft darstellen. Eine wesentliche Rolle bei diesem Zugang zu synthetischen Sandelriechstoffen hat dabei die Tatsache gespielt, daß Campholenaldehyd (B), der den referierten Verbindungen zugrundeliegende Synthesebaustein, leicht aus alpha-Pinen, einem Naturstoff, zugänglich ist. 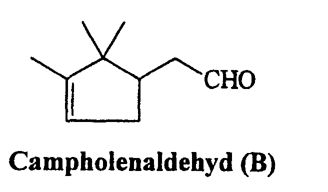 2-Methyl-4-(2,2,3-trimethyl-cyclopent-3-en-1-yl)-but-2-en-1-ol, fortan abkürzend als "Sandelol" (A) bezeichnet, ist ein begehrter Riechstoff mit ausgeprägtem Sandelduft. Die Strukturformel von Sandelol ist wie folgt: 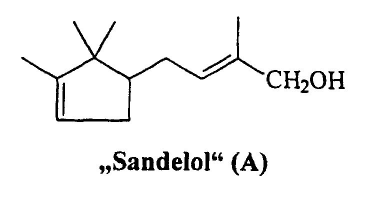 Sandelol ist dem Fachmann bereits seit längerer Zeit bekannt. So lehrt bereits die deutsche Offenlegungsschrift **DE-A-19 22 391,** daß Sandelol erhalten wird, indem man zunächst Campholenaldehyd mit Propionaldehyd in Gegenwart stark basischer Katalysatoren wie Alkalialkoholaten oder Alkalihydroxiden im Sinne einer Aldolkondensation umsetzt und den dabei erhaltenen Rs,^{MD}-ungesättigten Aldehyd in einem weiteren Schritt zum entsprechenden Alkohol - eben dem Sandelol - reduziert. Die Reduktion kann dabei mit komplexen Metallhydriden (etwa Lithiumaluminiumhydrid) oder mit Aluminiumalkoholaten nach der klassischem Methode von Meerwein-Ponndorf erfolgen.

**JP-A2-55/036423** (zitiert nach Chem. Abstr. 93/094886p) beschreibt ein spezielles Verfahren zur Herstellung von Sandelol. Demnach wird im ersten Schritt - völlig analog zur Lehre der zitierten DE-A-19 22 391 Campholenaldehyd (B) in Gegenwart von Natriumhydroxid als basischem Katalysator mit Propionaldehyd umgesetzt. Der bei dieser gemischten Aldolkondensation entstehende Rs,^{MD}-ungesättigte Aldehyd (C) wurde in einer Ausbeute von 73,5% isoliert. 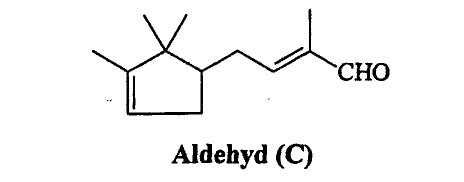 In einem weiteren Schritt wurde schließlich dieser ungesättigte Aldehyd (C) mit Al[OCH(CH₃)₂]₃ zum entsprechenden Sandelol (A) reduziert. Die dabei erzielte Ausbeute wird mit 85% angegeben.

Aus der jüngeren Literatur ist bekannt, daß saure Hilfsreagentien wie Trifluoressigsäure in der Lage sind, die klassische Meerwein-Ponndorf-Reduktion von Carbonylverbindungen mit Aluminiumalkoholen zu beschleunigen, vergleiche zum Beispiel in **Tetrahedron Letters 1995, 36 (20), 3571-3572 sowie 1995, 36 (28), 5085-5088.**

### Beschreibung der Erfindung

Das aus JP-A2-55/036423 bekannte Verfahren zur Herstellung des Sandelols (A) vermag jedoch im Hinblick auf Ausbeute und Wirtschaftlichkeit nicht vollständig zu befriedigen. Es bestand daher Bedarf nach einem verbesserten Verfahren zur Herstellung der Verbindungen (A).

Es wurde nun überraschend gefunden, daß sich "Sandelol" (A) 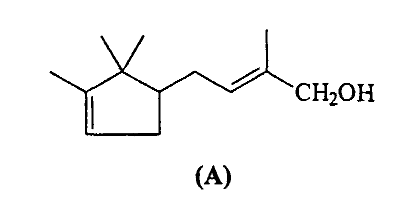 in hohen Ausbeuten herstellen läßt, indem man zunächst Campholenaldehyd und Propionaldehyd in üblicher Weise einer Aldolkondensation unterwirft und das dabei erhaltenen Reaktionsgemisch, das überwiegend den Aldehyd (C) 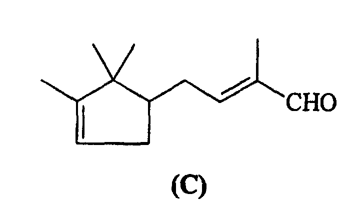 enthält, anschließend einer Reduktion mit Aluminiumalkoholaten nach Meerwein-Ponndorf unterwirft, wobei man diese Reduktion in Gegenwart basicher Aminverbindungen durchführt.

Gegenstand der vorliegenden Erfindung ist daher ein Verfahren zur Herstellung von 2-Methyl-4-(2,2,3-trimethyl-cyclopent-3-en-1-yl)-but-2-en-1-ol (A), 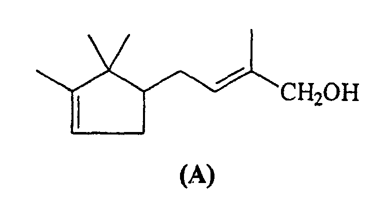 wobei man zunächst Campholenaldehyd und Propionaldehyd in üblicher Weise im Sinne einer Aldolkondensation miteinander umsetzt und das dabei erhaltenen Reaktionsgemisch, das überwiegend den Aldehyd (C) 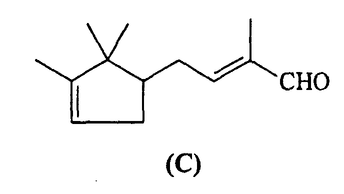 enthält, anschließend einer Reduktion mit Aluminiumalkoholaten nach Meerwein-Ponndorf unterwirft, wobei man diese Reduktion in Gegenwart basicher Aminverbindungen durchführt.

Das erfindungsgemäße Verfahren hat gegenüber dem herkömmlichen Stand der Technik den Vorteil, daß Zwischen- und Wertprodukte in hoher Reinheit und in nahezu quantitativer Ausbeute erhalten werden.

Die Aldolkondensation, also die erste Stufe der Sandelol-Synthese kann in inerten organischen Lösungsmitteln durchgeführt werden. Hierzu eignen sich insbesondere unpolare Lösungsmittel, die mit Wasser ein Azeotrop bilden. Beispiele für geeignete Lösungsmittel sind Toluol, Xylol, Benzol, Cyclohexan und Methylcyclohexan.

In einer speziellen Ausführungsform der Erfindung setzt man zur Katalyse der Aldolkondensation Ammoniumsalze einer organischen Säure ein. Die Art der Säure ist dabei an sich nicht kritisch. Ebenso wenig spielt es eine Rolle ob das Ammoniumsalz als solches eingesetzt wird oder ob es während der Reaktion - etwa aus einem Amin und einer organischen Säure - in situ gebildet wird. Beispiele für geeignete Ammoniumsalze sind: Benzyltrimethylammoniumhydroxid, Piperidinylacetat, Pyrrolidiniumacetat, Ammoniumacetat, Dimethylammoniumpyridinylacetat, Morpholinacetat, Lewatit 11600 (mit Essigsäure aktiviert), Piperidinylformiat, N,N-Tetraacetylethylendiamin, N,N-Diacetylethylendiamin, Dibutylammoniumacetat und Piperidinylpropionat. Die Konzentration des Katalysators liegt vorzugsweise im Bereich von 0,001 bis 20 Mol-%, insbesondere 0,5 bis 10 Mol-% - bezogen auf Campholenaldehyd (B).

Propionaldehyd wird vorzugsweise in 2,5- bis 10-molarem Überschuß - bezogen auf Campholenaldehyd (B) - eingesetzt. Insbesondere setzt man Propionaldehyd in 2,5 bis 3,5-molarem Überschuß ein.

Das erfindungsgemäße Verfahren kann bei Temperaturen von 20 bis 150 °C durchgeführt werden. Dabei ist es besonders bevorzugt, eine Reaktionstemperatur im Bereich von 40 bis 120 °C einzustellen.

In der zweiten Stufe der Sandelol-Synthese wird die Reduktion mit Aluminiumalkoholaten nach Meerwein-Ponndorf in Gegenwart basischer Aminverbindungen durchgeführt. Besonders bevorzugt ist dabei der Einsatz von Aluminiumisopropylat und/oder -butylat.

Beispiele für geeignete basische Aminoverbindungen sind Alkyl-, Dialkyl- und Trialkylamine, ferner Ethanolamine und -diamine, Pyrrole, Pyrrolidine und Piperidine. Es hat sich gezeigt, daß durch die Gegenwart der Amine, die vorzugsweise in Mengen von 0,001 bis 0,5 mol bezogen auf 1 mol des zu reduzierenden Aldehyds (C) eingesetzt werden, die Menge des Aluminiumalkoholates deutlich reduziert werden kann: Während Aluminiumalkoholate bei der Meerwein-Ponndorf-Reduktion üblicherweise in Mengen von mehr als 0,2 mol (bezogen auf den zu reduzierenden Aldehyd) eingesetzt werden, genügen bei dem Verfahren gemäß der vorliegenden Erfindung Aluminiumalkoholat-Mengen von weniger als 0,1 mol (bezogen auf den zu reduzierenden Aldehyd).

Das erfindungsgemäße Verfahren hat ferner folgende Vorteile: verbesserte Ausbeuten; geringerer Aufwand beim Abtrennen der Aluminiumalkoholate; höhere Reinheit des Produktes.

Die folgenden Beispiele dienen der Erläuterung der Erfindung und sind nicht einschränkend zu verstehen.

### Beispiele

### 1. Verwendete Substanzen

### 1.1. für die Aldolkondensation

alpha-Campholenaldehyd: 85%-ig (Fa. Glidco)
Propionaldehyd: 98%-ig (Fa. Riedel-de Häen)

### 1.2. für die Reduktion des ungesättigten Aldehyds

Aluminiumtriisopropylat: Aluminiumisopropanolat (> 98 %ig), Fa. Fluka AG

### 2. Durchführung der Reaktionen

### 2.1. Aldolkondensation

### Beispiel 1

In einem 2-Liter-Dreihalskolben wurden 12 g Natriumstücke nach und nach in 500 ml wasserfreiem Methanol unter Rühren gelöst und mit einem Eisbad auf ca. 15 °C gekühlt. Anschließend wurden unter Stickstoff und Rühren ein Gemisch aus 152 g (0,85 mol) alpha-Campholenaldehyd, 72,5 g (1,25 mol) Propionaldehyd und 50 ml Methanol in 1,5 Std. bei 18 °C (Innentemperatur) über einen Tropfrichter zudosiert. Anschließend wurden noch 1,5 Std. weiter gerührt: Nach 3 Std. Reaktion betrug der pH der Mischung 12,9. Es wurde mit ca. 50 ml konzentrierter HCl unter Rühren und Eiswasserkühlung neutralisiert.

Zur Aufarbeitung wurde die Mischung zweimal mit je 500 ml Petrolether (40/60) extrahiert und die vereinigten organischen Phasen zweimal mit je 200 ml entmineralsiertem Wasser gewaschen, über Nacht über Natriumsulfat getrocknet und nach Abfiltrieren am Rotavapor eingeengt. Die Auswaage betrug 175 g. Die gaschromatographische Analyse ergab folgende Zusammensetzung (Flächen-%): 17,3 % Campholenaldehyd, 5,1 % freier Propionaldehyd und 50,0 % Aldol-Kondensationsprodukt. Der Anteil der Mischung an diesem Aldol-Kondensationsprodukt konnte anschließend durch Fraktionierung im Hochvakuum unter Verwendung einer Vigreux-Kolonne auf > 85 % gesteigert werden.

### 2.2. Reduktion

In einem 2-Liter-Dreihalskolben mit Destillationsaufsatz wurden 408 g (ca. 2 mol) ungesättigter Aldehyd aus der Aldolkondensation (Reinheit > 85 %) unter Stickstoffatmosphäre bei ca. 70 ° C Siedetemperatur und starkem Rühren zu einer Mischung aus 41 g (ca. 0,2 mol) Aluminiumtriisopropylat, 6 g Diisopropylamin und 600 g wasserfreiem Isopropanol innerhalb von 1 Stunde zudosiert. Dabei wurde kontinuierlich ein Gemisch aus Aceton, Isopropanol und Diisoproylamin abdestilliert und von Zeit zu Zeit durch Zugabe von je 300 g frischem wasserfreiem Isopropanol ersetzt. Nach 8-stündiger Reaktionszeit war im Destillat über das Phenylhydrazon kein Aceton mehr fällbar. Das Lösungsmittelgemisch wurde über Kopf abdestilliert und der Rückstand auf Raumtemperatur gekühlt.

Zur Aufarbeitung wurde das Produktgemisch durch zweimaliges Waschen mit je 500 ml 10 %iger Natronlauge bei Temperaturen zwischen 50 und 70 °C, um die Phasentrennung zu verbessern, von Aluminiumsalzen befreit. Es wurde zweimal mit je 500 ml entmineralisiertem Wasser nachgewaschen. Die organischen Phase wurde roh der Fraktionierung zugeführt. Es wurde im Hochvakuum an einer Drehbandkolonne fraktioniert. Der Hauptlauf wurde gaschromatographisch charakterisiert (Flächen-%): 90,5 Sandelol (A); 4,7 % Edukt; 2,9 % Nebenprodukte.

## Patentansprüche

1. Verfahren zur Herstellung des Sandel-Riechstoffes 2-Methyl-4-(2,2,3-trimethyl-cyclopent-3-en-1-yl)-but-2-en-1-ol (A), 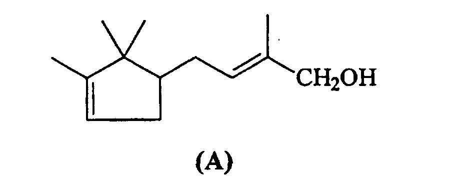 wobei man zunächst Campholenaldehyd und Propionaldehyd in üblicher Weise im Sinne einer Aldolkondensation miteinander umsetzt und das dabei erhaltenen Reaktionsgemisch, das überwiegend den Aldehyd (C) 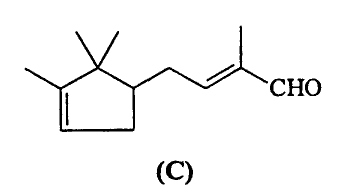 enthält, anschließend einer Reduktion mit Aluminiumalkoholaten nach Meerwein-Ponndorf unterwirft, **dadurch gekennzeichnet**, daß man die Reduktion nach Meerwein-Ponndorf in Gegenwart basischer Aminverbindungen durchführt.

## Claims

1. A process for the production of the sandalwood fragrance 2-methyl-4-(2,2,3-trimethylcyclopent-3-en-1-yl)-but-2-en-1-ol (A): 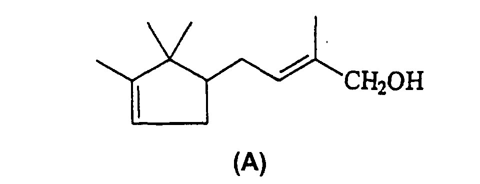 in which campholene aldehyde and propionaldehyde are conventionally reacted with one another by aldol condensation and the reaction mixture obtained, which mainly contains the aldehyde (C): 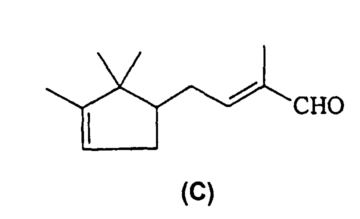 is subsequently subjected to Meerwein-Ponndorf reduction with aluminium alcoholates, **characterized in that** the Meerwein-Ponndorf reduction is carried out in the presence of basic amine compounds.

## Revendications

1. Procédé pour la préparation du 2-méthyl-4-(2,2,3-triméthylcyclopent-3-én-1-yl)but-2-én-1-ol (A) 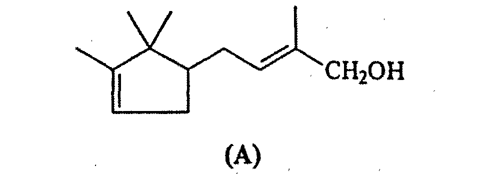 dans lequel on fait d'abord réagir l'un avec l'autre du campholénaldéhyde et du propionaldéhyde, à la manière usuelle, dans le sens d'une condensation en aldol, et on soumet ensuite le mélange réactionnel ainsi obtenu, qui contient essentiellement l'aldéhyde (C) 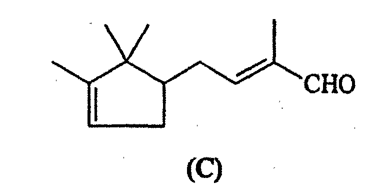 à une réduction avec des alcoolates d'aluminium, selon Meerwein-Ponndorf,
**caractérisé en ce qu**'
on effectue la réduction de Meerwein-Ponndorf en présence de composés amino basiques.
